# EUROPEAN PATENT APPLICATION

(11) **EP 2 540 209 A1**
(43) Date of publication of application: **02.01.2013**
(21) Application number: 11746985.8
(22) Date of filing: 07.02.2011
(51) Int. Cl.: A61B 1/00

(54) **ENDOSCOPE INSERTION AID**

(30) Priority: 24.02.2010 JP 2010038131
(71) Applicant: National University Corporation Kagawa University, Takamatsu-shi, Kagawa 760-8521 (JP); KABUSHIKI KAISHA TOP, Adachi-ku, Tokyo 120-0035 (JP)
(72) Inventor: MORI, Hirohito, Kita-gun Kagawa 761-0793 (JP)
(74) Representative: Schön, Christoph
(86) International application number: PCT/JP2011/000652
(87) International publication number: WO 2011/105012

(57) **Abstract**

An endoscope insertion aid capable of preventing deterioration of operability of an endoscope by an endoscopist even when a posture of a patient receiving examination changes. The aid 10 which guides the endoscope to a mouthpiece M held by a mouth of the patient to whom the endoscope is inserted is equipped with a body section 11 equipped with a bent through-hole having an inner diameter capable of inserting the endoscope, and a holding section 15 which fixes the body section 11 so as not to move when the endoscope is inserted to the body section 11. By arranging the body section 11 so that one end opening of the through-hole opposes a through-hole Mh of the mouthpiece M, a shaft S of the endoscope may be inserted to the mouthpiece M held by the patient's mouth even when the endoscope is in a position different from the direction the mouth of the patient is facing. By doing so, the endoscopist is capable of operating the endoscope from a given location regardless of the patient's posture, it becomes possible to prevent deterioration of operability of the endoscope by the endoscopist by the posture of the patient.

## Description

### Technical Field

The present invention relates to an endoscope insertion aid.

### Background Art

Conventionally, there is performed an examination or a treatment inside a stomach using a peroral endoscope (a so-called gastroscope, hereinafter simply referred to as an endoscope).
The endoscope is equipped with an optical fiber for transmitting and receiving images and illumination light, a pipe for inserting and ejecting equipments for the examination and treatment, and for supplying and suctioning air and liquid, and a shaft (a fiberscope) mounted with a wire for moving a leading tip thereof and the like. By inputting the shaft into the interior of the stomach, the examination or the treatment inside the stomach with the endoscope is made possible.

The examination or the treatment using such endoscope is used as an extremely effective means for treatment of patients visiting emergency department.
For example, when a patient visiting the emergency department is diagnosed as a gastrointestinal hemorrhage (a gastric ulcer, a duodenal ulcer, a stomach cancer, an esophageal varix, a gastric varix, and the like) after diagnosis by an emergency physician, an emergency endoscopy examination and treatment is performed by a digestive endoscopist (a physician specialized in endoscope, hereinafter simply referred to as an endoscopist).
With the current endoscopic technique, even when the gastrointestinal hemorrhage has occurred, it becomes possible to restrain hemorrhage almost for certain if the location of hemorrhage is identified. Therefore, it is important to promptly perform identification and determination of the location of hemorrhage in promptly treating (restrain hemorrhage of) the patient.

In the case where the gastrointestinal hemorrhage has occurred, it is easy to identify the location of hemorrhage if the location of hemorrhage is exposed.
However, when the posture of the patient is held at a left lateral recumbent position which is a basic posture of the endoscopy, a gastric body to a fundus becomes the direction of gravity. As such, in the case where the gastrointestinal hemorrhage has occurred, blood from hemorrhage is accumulated at this location. In the case where the location of hemorrhage exists within the gastric body to the fundus, the location of hemorrhage is covered by the blood, and it is not possible to identify the location of hemorrhage with the endoscope in such state.

If the accumulated blood is not clotted, it becomes possible to secure the field of view and identify the location of hemorrhage, by suctioning and removing the blood by suctioning operation of the endoscope.
However, if some time has lapsed since hemorrhage, and the blood had clotted to a clot or to a nearly gelled state, there is a possibility that the clotted blood cannot be suctioned and removed by the endoscope. In the case where the location of hemorrhage is covered by the clotted blood, the location of hemorrhage cannot be identified.
In this case, in order to identify and determine the location of hemorrhage, the posture of the patient is changed so as to move the clot inside the stomach. Specifically, in order to move the clot inside the stomach at the left side (for example, the clot existing within the gastric body to the fundus) to the right side, the patient is changed to a right lateral recumbent position.

From the state where the endoscopy is performed at the left lateral recumbent position, when the patient is changed to the right lateral recumbent position, the patient takes a posture showing his/her back to the endoscopist, so that the mouth of the patient faces opposite direction with respect to the endoscopist. Then, the endoscope cannot be inserted into the patient in the state where the examination is performed in the left lateral recumbent position, it becomes necessary for the endoscopist to move from the left side to the right side of the patient, and also to move the endoscope body and the light source to the right side of the patient.

In the endoscopy in the left lateral recumbent position (the standard posture), the endoscopist inserts the endoscope while holding an endoscope operation unit (various buttons and levers) with his/her left hand, and while holding a shaft of the endoscope (tube portion) with his/her right hand. At this time, the direction is set by operating the vertical and horizontal direction of a leading end of the endoscope with the lever operation by the left hand. Then, the shaft is inserted by the right hand, so as to move the leading end of the shaft from an esophagus to the stomach.

On the other hand, in the endoscopy in the right lateral recumbent position, in the method of inserting the endoscope by the endoscopist, the endoscope must be operated in a completely different method from the left lateral recumbent position. Specifically, the endoscopist must operate the endoscope while holding an angle lever in the right hand and the shaft in the left hand.
The endoscope may be operated while holding the angle lever in the left hand and the shaft in the right hand, similar to the case of the left lateral recumbent position, however, in this case, the treatment must be performed with a mirror-reversed image. This is a state extremely difficult for the endoscopist to operate the endoscope.
That is, in the case where the right lateral recumbent position which is opposite to the left lateral recumbent position as the standard posture is taken, the operation of the endoscope by the endoscopist becomes extremely difficult, so that extra time becomes necessary for identifying the location of hemorrhage and restraining the hemorrhage, and also the accuracy thereof drops.

General status of the emergency patient with a hematemesis and the like suspected of the gastrointestinal hemorrhage, depends on hemorrhage volume. In the case of profuse hemorrhage, the general status is often not good, such as blood pressure reductions, deteriorated aspiration, disordered consciousness from severe anemia from hemorrhage. Therefore, in the emergency hemorrhage restraining treatment using endoscope, prompt start of treatment and prompt restraining of hemorrhage is necessary.
In the emergency hemorrhage restraining treatment using endoscope, it is required to promptly perform the examination and treatment with endoscope in the above-mentioned right lateral recumbent position, and therefore it is necessary to improve the operability of the endoscope by the endoscopist so that the similar examination and treatment as in the left lateral recumbent position may be performed in the right lateral recumbent position.

Conventionally, as an aid which facilitates the examination or the treatment by endoscope, a mouthpiece or a guide tube and the like are developed and used (for example, refer to Patent Documents 1 through 4).
However, the conventional mouthpiece is merely provided in order to prevent the operation of the fiberscope from becoming impossible by the patient biting the fiberscope.
Further, the conventional guide tube is merely used for surely guiding a portion inserted inside a body of a human.
That is, in the case where the posture of the patient is changed, both equipments do not prevent deterioration of the operability of the endoscope by the endoscopist.

Currently, there is no aid which improves the operability of the endoscope by the endoscopist in the case where the patient is different (the right lateral recumbent position) from the standard posture (the left lateral recumbent position), and there is a need for the development of such aid.

### Prior Art Reference

### Patent Document

Patent Document 1: Japanese Patent Laid-open No. 2000-229062
Patent Document 2: Japanese Patent Laid-open No. 2008-289592
Patent Document 3: Japanese Patent Laid-open No. 2008-289520
Patent Document 4: Japanese Patent Laid-open No. 2007-75284

### Disclosure of the Invention

### Problems to be solved by the Invention

In view of the above-mentioned circumstances, the present invention aims at providing an endoscope insertion aid which is capable of preventing deterioration of the operability of the endoscope by the endoscopist, even when the posture of the patient receiving the examination changes.

### Means for solving the Problems

An endoscope insertion aid of a first aspect of the invention is an aid which supports an endoscope inserted to a mouthpiece held in a mouth of a patient, at outside a mouth cavity, characterized in that the endoscope insertion aid is equipped with a body section equipped with a through-hole having an inner diameter capable of being inserted with the endoscope, and the body section is capable of holding the endoscope in a bent state, in a state where the endoscope is inserted into the through-hole of the body section and thereafter inserted to the mouthpiece.
The endoscope insertion aid of a second aspect of the invention is characterized in that, in the first aspect of the invention, the body section is equipped with a holding section which fixes the body section so as not to move.

### (Fixed type)

The endoscope insertion aid of a third aspect of the invention is characterized in that, in the second aspect of the invention, the through-hole formed on the body section is bent, and the holding section is for fixing the body section so as not to move by a force applied from the endoscope when the endoscope is inserted into the through-hole of the body section.

The endoscope insertion aid of a fourth aspect of the invention is characterized in that, in the first, the second, or the third aspect of the invention, the body section is equipped with a guide pipe which is a tubular member and which is bent between one end and the other end thereof.

The endoscope insertion aid of a fifth aspect of the invention is characterized in that, in the third or the fourth aspect of the invention, the body section is equipped with the guide pipe, and a supporting member which supports a force applied from the endoscope to the guide pipe when the endoscope is inserted into the guide pipe, and the supporting member is equipped with a concaved supporting curved surface which supports the force applied from the endoscope to the guide pipe.

The endoscope insertion aid of a sixth aspect of the invention is characterized in that, in the third, the fourth or the fifth aspect of the present invention, the guide pipe is a tubular member formed in a U-shape.

### (Movable type)

The endoscope insertion aid of a seventh aspect of the invention is characterized in that, in the first or the second aspect of the invention, the body section is equipped with a shell member formed with the through-hole and which is capable of deforming so that a central axis of the through-hole becomes a bent state between one end and the other end of the through-hole, and a posture holding means for holding the posture of the shell member.

The endoscope insertion aid of an eighth aspect of the invention is characterized in that, in the seventh aspect of the invention, the shell member is formed from a plurality of tubular members, and a plurality of the tubular members are connected so that central axes of all tubular members are positioned within an identical plane, and, adjacent tubular members are mutually flexible in the state where the central axes of all tubular members are positioned within the identical plane.

The endoscope insertion aid of a ninth aspect of the invention is characterized in that, in the seventh or the eighth aspect of the invention, the tubular member is equipped with a pair of wall members having a tubular inner surface, and a pair of the wall members are configured so as to be capable of approaching and departing from one another, and so that the through-hole is formed between the two in the state where the two are approaching one another, and the endoscope is capable of being arranged between the two when the two are departing from one another.

### (Mask type)

The endoscope insertion aid of a tenth aspect of the invention is characterized in that, in any one of the second through the ninth aspect of the invention, the body section is a tubular member, the holding section is equipped with a mouthpiece to which one end of the body section is mounted, a mask plate which is fixed with the mouthpiece, and which is mounted so as to cover a face of a patient, and a fixing means for fixing the mask plate to a head of the patient, and the mask plate is formed from a material having same or surpassing intensity as the material of the mouthpiece.

The endoscope insertion aid of an eleventh aspect of the invention is characterized in that, in the tenth aspect of the invention, one end of the body section is removable and attachable with respect to the mouthpiece of the holding section.

### Effect of the Invention

According to the first aspect of the invention, by inserting the shaft of the endoscope to the mouthpiece after inserting the same to the through-hole formed in the body section, it becomes possible to hold the shaft of the endoscope to the bent state. By adjusting the bentness of the shaft of the endoscope, the endoscopist may operate the endoscope from a given location, regardless of the posture of the patient, so that it becomes possible to prevent the deterioration of the operability of the endoscope by the endoscopist with the posture of the patient.
According to the second aspect of the invention, the force applied from the shaft of the endoscope to the body section when the shaft of the endoscope is inserted into the through-hole formed in the body section may be supported by the holding section, it becomes possible to prevent the body section from moving by the force.

### (Fixed type)

According to the third aspect of the invention, the through-hole formed in the body section is bent, and also, the body section is held by the holding section so as not to move even when the endoscope is inserted. Therefore, by arranging the body section so that the one end opening of the through-hole opposes the through-hole of the mouthpiece, the shaft of the endoscope may be inserted into the mouthpiece held in a mouth of the patient, even when the endoscopist is in a position different from the direction which the mouth of the patient is facing. Then, it becomes possible for the endoscopist to operate the endoscope from a given location regardless of the posture of the patient, so that it becomes possible to prevent deterioration of the operability of the endoscope by the endoscopist with the posture of the patient.

According to the fourth aspect of the invention, by inserting the shaft of the endoscope into the guide pipe, and move the shaft along the guide pipe, it becomes possible to guide the leading end of the shaft to the mouthpiece held in the mouth of the patient. Further, the through-hole is formed by the tubular member, so that the body section may be a simple structure.

According to the fifth aspect of the invention, by arranging the body section so that the supporting curved surface opposes the patient, the force applied from the shaft of the endoscope may be held by the supporting curved surface of the supporting member, so that the intensity of the guide pipe may be reduced. And, if the member having flexibility is used as the guide pipe, the relative position between the one end opening of the through-hole and the through-hole of the mouthpiece held in the mouth of the patient may be adjusted easily.

According to the sixth aspect of the invention, the endoscope may be operated similarly to the patient in the left lateral recumbent position, even when the patient is in the right lateral recumbent position, so that it is possible to prevent deterioration of the operability of the endoscope by the endoscopist.

### (Movable type)

According to the seventh aspect of the invention, by adjusting the bentness of the shell member and thereafter fixing the bentness of the shell member by the posture holding means, it becomes possible to make the through-hole of the shell member as the through-hole having a predetermined bentness. Therefore, by making the body section so that the one end opening of the through-hole opposes the through-hole of the mouthpiece, and the other end opening faces the endoscopist, it becomes possible for the endoscopist to operate the endoscope from a given location regardless of the posture of the patient. Therefore, it becomes possible to prevent deterioration in the operability of the endoscope by the endoscopist from the posture of the patient. Further, if the shell member is attached to the endoscope, the endoscope may be bent according to the posture change, even when the posture of the patient is changed while the endoscope is inserted, by deforming the shell member. By doing so, it is not necessary to perform insertion of the endoscope each time the posture of the patient is changed, so that it becomes possible to promptly perform the examination and treatment with the endoscope.

According to the eighth aspect of the invention, the through-hole of the shell member may be made to a bent state, if the adjacent tubular members are flexed. Further, the shell member is formed merely by connecting the tubular members mutually flexibly, so that the structure of the shell member becomes simple, and the rigidity of each tubular member may be increased.

According to the ninth aspect of the invention, by departing the pair of wall members of the tubular member from each other, the shaft of the endoscope may be inserted inside the through-hole of the shell member, so that it becomes possible to accommodate the shaft of the endoscope inside the through-hole of the shell member, while the shaft of the endoscope is inserted inside the patient. By doing so, in the case where there is a necessity of changing the posture of the patient after starting the examination or the treatment, it becomes possible to change the posture of the patient while the endoscope is inserted inside the patient, by attaching the shell member.

### (Mask type)

According to the tenth aspect of the invention, the body section is fixed to the mouthpiece, so that it becomes possible to surely guide the shaft of the endoscope to the mouth of the patient. Further, the mask plate with high intensity is attached to the face of the patient, so that the force applied from the shaft of the endoscope to the body section may be supported by the jawbone or the like. Therefore, even during examination by the endoscope, the body section may be held in a stable state.

According to the eleventh aspect of the invention, the body section is removable with respect to the mouthpiece, so that the holding section may be attached to the patient in the normal state, and the body section may be used only in the case where it is necessary. By doing so, when the posture of the patient is changed, the examination or the treatment after posture change of the patient may be performed only by removing and attaching the body section, the posture changing operation is made easy, and the examination and the treatment may be started promptly after the posture change.

### Brief Description of the Drawings

[Fig. 1] A rough explanatory view of a situation where an endoscopy is performed to a patient in a right lateral recumbent position using an endoscope insertion aid 10 of the present embodiment.
[Fig. 2] Fig. 2 (A) is a rough explanatory view of a situation where the endoscopy is performed to the patient in the right lateral recumbent position using the endoscope insertion aid 10 of another embodiment, and Fig. 2(B) is an enlarged rough explanatory view of a supporting member 13.
[Fig. 3] A rough front view of the patient wearing an endoscope insertion aid 20 of another embodiment.
[Fig. 4] A rough side view of the patient wearing the endoscope insertion aid 20 of another embodiment.
[Fig. 5] A rough explanatory view of a situation where the endoscopy is performed to the patient in a left lateral recumbent position.

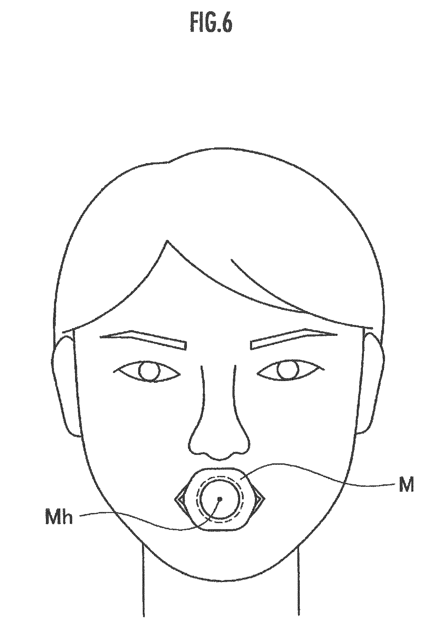

[Fig. 6] A rough front view of the patient wearing a mouthpiece M.
[Fig. 7] A rough explanatory view of a shell member 14, and Fig. 7(A) is a state where the shell member 14 is extended, Fig. 7(B) is a state where the shell member 14 is bent, and Fig. 7(C) is a rough explanatory view of a tubular member 14a alone.

### Mode for Carrying Out the Invention

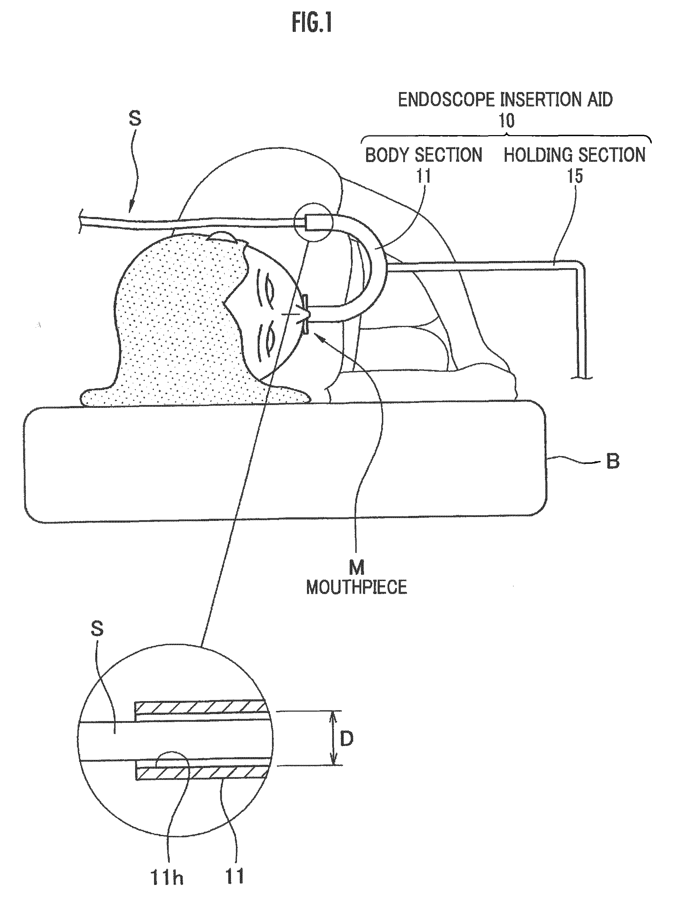

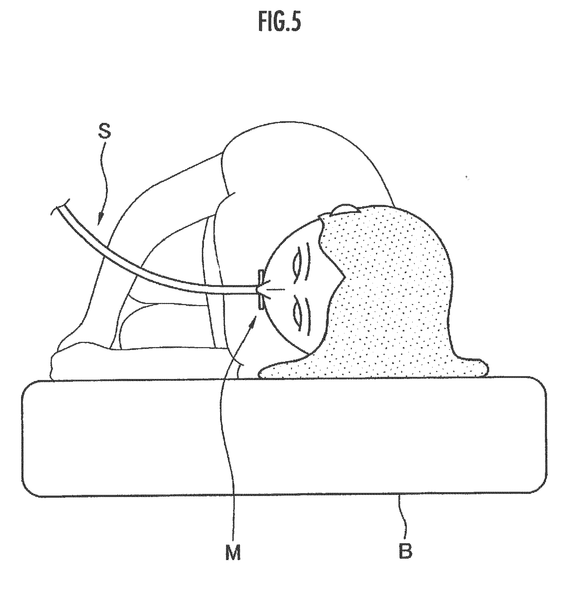

Next, explanation will be given on embodiments of the present invention on the basis of the accompanying drawings.
An endoscope insertion aid of the present invention is an aid used when performing examination and treatment by an endoscope inserted into a human body from a mouth, and has a characteristics that it makes it easier to perform the examination and the treatment using the endoscope to a patient in the state other than a left lateral recumbent position, such as a right lateral recumbent position or lying on a back.
The state of the right lateral recumbent position means a state where the patient is lying sideways with his/her right arm down (refer to Fig. 1). This state is a posture opposite to the left lateral recumbent position (refer to Fig. 5) that the patient normally takes when performing endoscopy, that is, the patient is lying sideways with his/her left arm down.

An endoscope insertion aid 10 of the present embodiment will be explained.
As is shown in Fig. 1, the endoscope insertion aid 10 of the present embodiment is equipped with a body section 11 and a holding section 15 connected to the body section 11.

First, the body section 11 is a member formed in a tubular shape, and is bent to a U-shape between one end and the other end thereof. The body section 11 is formed with a through-hole 11h which pass through between the one end and the other end in an axial direction thereof. That is, the body section 11 is formed with the through-hole 11h which passes between the one end and the other end thereof, and which is bent in a U-shape.
The through-hole 11h formed in the body section 11 is formed in a size so that the inner diameter D thereof is capable of inserting a shaft S of the endoscope. Specifically, the inner diameter D of the through-hole 11h is formed so that a clearance of approximately 3 to 5 mm is formed between a surface of the shaft S and an inner surface of the through-hole 11h when the shaft S is inserted. Currently, an outer diameter of the shaft S of the endoscope inserted into a human body from a mouth is roughly 9 to 12 mm, so that the inner diameter D of the through-hole 11h should be 12 to 17 mm.

The holding section 15 is a member for fixing the body section 11 so that it does not move. Specifically, the holding section 15 is a member for supporting a force applied to the body section 11 from the shaft S when the shaft S of the endoscope is inserted into the through-hole 11h of the body section 11, and for holding the body section 11 so that the same does not move by this force.
The structure of the holding section 15 is not limited, as long as it is capable of holding the body section 11 so that it does not move. For example, the holding section 15 may be configured from a rod-like member such as an angle with one end thereof fixed to the body section 11, and a publicly known fixing means for fixing the rod-like member to an examination table or an operating table.

Next, the use of the endoscope insertion aid 10 of the present embodiment will be explained.
First, in an ordinary endoscopy and the like, a patient holding a mouthpiece M in his/her mouth lies on a bed B in the left lateral recumbent position. Then, the endoscopist carries out the insertion of the shaft S and the examination, by operating the endoscope at the front of the patient in the left lateral recumbent position (Fig. 5).

If a region which cannot be examined in the left lateral recumbent position is found, then the patient changes its position from the left lateral recumbent position to the right lateral recumbent position after pulling out the shaft of the endoscope. Then, the endoscopist will be positioned at the back of the patient in the right lateral recumbent position.
When the patient takes the right lateral recumbent position, the body section 11 of the endoscope insertion aid 10 of the present embodiment is arranged so as to be positioned at a front of a face of the patient, and is fixed to the bed B or the like by the holding section 15. More specifically, the body section 11 is arranged so that the one end opening of the through-hole 11h opposes a through-hole Mh of the mouthpiece M held in the mouth of the patient.

When the body section 11 is arranged as is explained above, because the body section 11 is bent in the U-shape and the through-hole 11h thereof is also bent in the U-shape, the other end opening of the body section 11 is opened to the back of the patient in the right lateral recumbent position. That is, the other end opening of the body section 11 is facing the endoscopist positioned at the rear of the patient in the right lateral recumbent position.

When the endoscopist inserts the shaft S from the other end opening of the body section 11 in this state, the shaft S may be moved while being guided by the through-hole 11h bent in the U-shape, so that the shaft S may be guided to the one end opening of the body section 11. The one end opening of the body section 11 is arranged so as to oppose the through-hole Mh of the mouthpiece M held in the mouth of the patient, so that it becomes possible to insert the shaft S into the body of the patient through the through-hole Mh of the mouthpiece M (Fig. 1).

That is, by using the endoscope insertion aid 10 of the present embodiment, the endoscopist may perform examination and treatment to the patient in the right lateral recumbent position, in the same condition as the examination and treatment for the patient in the left lateral recumbent position. Stated otherwise, even when the patient is in the right lateral recumbent position, the endoscope may be operated in the same manner as in the state where the patient is in the left lateral recumbent position, so that it becomes possible to prevent deterioration of operability when the endoscopist is operating the endoscope.

Further, when the shaft S passes through the through-hole 11h of the body section 11, the shaft S must be bent along the shape of the through-hole 11h. Further, in the sate where the shaft S is arranged inside the through-hole 11h of the body section 11 (that is, during examination and treatment), the shaft S must be maintained in the state of being bent along the shape of the through-hole 11h. As such, a force for bending the shaft S and a reactive force of the force for bending the shaft S (a resilient force of the shaft S trying to become straight, and the like) is applied from the shaft S to the body section 11.
However, in the endoscope insertion aid 10 of the present embodiment, these reactive forces are supported by the holding section 15, so that it becomes possible to prevent the body section 11 from moving by the reactive force. That is, even in the state where the shaft S is inserted into the through-hole 11h of the body section 11, the body section 11 may be maintained in the state where the one end opening thereof is arranged so as to oppose the through-hole Mh of the mouthpiece M held in the mouth of the patient, it becomes possible to surely insert the shaft S to the mouthpiece M even to the patient in the right lateral recumbent position.

In order to make it easy for the shaft S of the endoscope to be inserted and to decrease the reactive force acting on the body section 11, it is preferable that a process for reducing a friction with the shaft S is performed to the inner surface of the through-hole 11h of the body section 11. For example, if a process for providing a layer of material having small coefficient of friction with the shaft S of the endoscope is performed on the inner surface of the through-hole 11h, such as a Teflon (registered trademark) process, it becomes possible to smoothly slide a tip of the shaft S and a side surface of the shaft S along the inner surface of the through-hole 11h and make the same move. If so, it becomes possible to perform operation of inserting the shaft S of the endoscope into the body of the patient easily and promptly, so that it becomes possible to shorten the time until starting the examination and treatment, and the time for the examination and treatment. Further, the force applied from the shaft S to the body section 11 is reduced, so that it becomes possible to reduce the intensity of the body section 11 and the holding section 15, and to reduce weight of the body section 11 and the holding section 15.

Further, the body section 11 may be formed so that the one end thereof is attachable to the mouthpiece M. For example, if an outer diameter of the one end of the body section 11 is made slightly smaller than the through-hole Mh of the mouthpiece M, it becomes possible to insert the one end into the through-hole Mh of the mouthpiece M. By doing so, it becomes possible to prevent the relative position of the body section 11 and the mouthpiece M from misaligning during examination and treatment with endoscope.
Especially, for the aim of preventing the misalignment of the body section 11 and the mouthpiece M, the mouthpiece M may be provided at one end of the body section 11.

### (Other shape of the body section 11)

In the above-mentioned example, an explanation is given on the case where the body section 11 is a member formed in tubular shape bent in the U-shape. However, the body section 11 does not have to be the U-shape, and the proportion of bending of the body section 11 may be selected variously. The body section 11 may be bent into an L-shape, bent into an intermediate shape between the U-shape and the L-shape, bent into a bent angle smaller than the L-shape, and the like.
For example, in the case of a patient who could only lie on his/her back, the shaft S of the endoscope must be inserted into the patient lying on his/her back. However, if the body section 11 is bent into the L-shape, the endoscopist may operate the endoscope in the same manner as with the patient in the left lateral recumbent position, even to the patient lying on his/her back.
Similarly, by using the body section 11 suitable to the posture of the patient, the endoscopist may operate the endoscope in the same manner as with the patient in the left lateral recumbent position, regardless of the posture of the patient.

In the above-mentioned example, explanation is given to the case where the body section 11 is a member formed into a tubular shape. However, the body section should only have a bent through-hole, and is not particularly limited. For example, it may be a block-shaped member formed with a through-hole, if it is capable of arranging an another end opening of the through-hole in a position opposing the position where the endoscopist is normally operating the endoscope in the state of the left lateral recumbent position, in the state where one end opening of the through-hole is arranged so as to oppose the through-hole Mh of the mouthpiece M held in the mouth of the patient.
However, forming the body section 11 from a tubular member is preferable from the viewpoint of making the body section 11 into a simple structure.

### (Other embodiments)

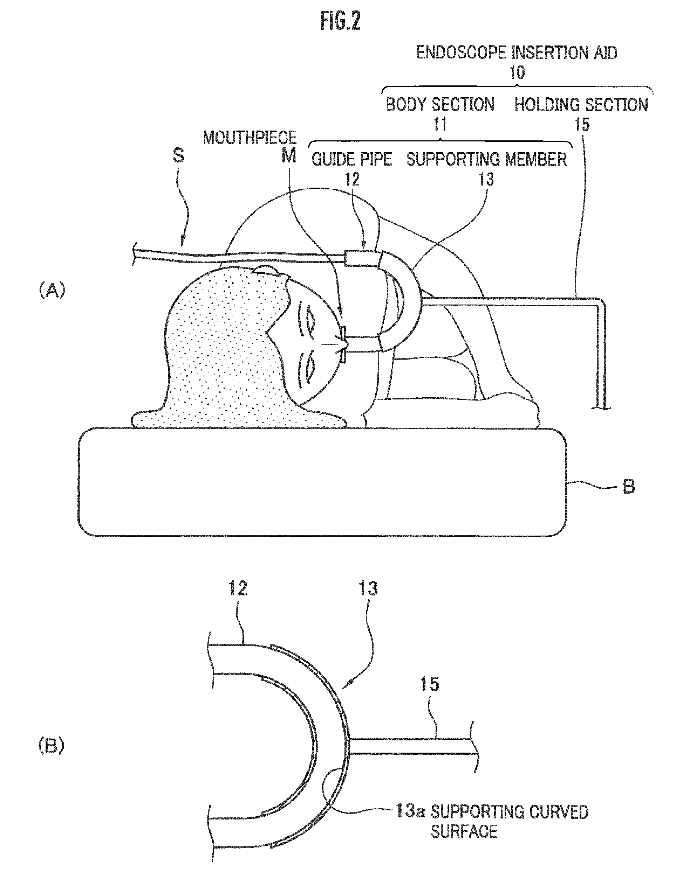

The material of the above-mentioned body section 11 is not particularly limited. The material may be the one having intensity to an extent that it does not break from the reactive force applied from the shaft S of the endoscope, and is not particularly limited.
However, the reactive force applied from the shaft S becomes large at the portion in which the shaft S is bent, so that the body section 11 may be configured from a guide pipe 12 having bent through-hole, and a supporting member 13 which supports the portion of the guide pipe 12 which is bent, as is shown in Fig. 2. For example, as is shown in Fig. 2, such supporting member 13 has, as the supporting member 13, the one having the through-hole for accommodating the guide pipe 12, and the through-hole has a concaved supporting curved surface 13a may be used.

When using such supporting member 13 in the examination and treatment, the body section 11 is arranged so that the supporting curved surface 13a of the supporting member 13 is in the state of being concave from the side of the patient holding the mouthpiece M in the mouth. Thereafter, the guide pipe 12 is arranged so that an outer surface thereof contacts the supporting curved surface 13a. For example, in the case where a member having flexibility (for example, a bellows tube) is used as the guide pipe 12, the guide pipe 12 is arranged in a state of being bent along the supporting curved surface 13a.
If so, it becomes possible to make the supporting member 13 to support the force applied to the guide pipe 12 from the shaft S of the endoscope, and to reduce the intensity of the guide pipe 12. That is, the intensity of the guide pipe 12 does not have to be the intensity capable of supporting the force applied from the shaft S. By doing so, a degree of freedom of the member to be used as the guide pipe 12 and the material thereof becomes higher, and it becomes possible to reduce weight and suppress manufacturing cost of the body section 11.
If a member having flexibility is used as the guide pipe 12, it becomes possible to finely tune a relative position between one end of the guide pipe 12 and the mouthpiece M held by the patient in the mouth, and a relative position between an another end of the guide pipe 12 and the endoscopist, so that it becomes possible to promptly start the examination and the treatment using the endoscope insertion aid 10 of the present embodiment.

### (Movable body section)

Further, in the above-mentioned supporting member 13, in the case where the patient changed his/her posture, in the case where the bentness state of the shaft S of the endoscope must be changed, the guide pipe 12 and the supporting member 13 must be changed. That is, each time the patient changes his/her posture, the shaft S of the endoscope must be taken ouf from the patient, and the shaft S must be inserted again into the patient after inserting the shaft S into another guide pipe 12 and the supporting member 13.

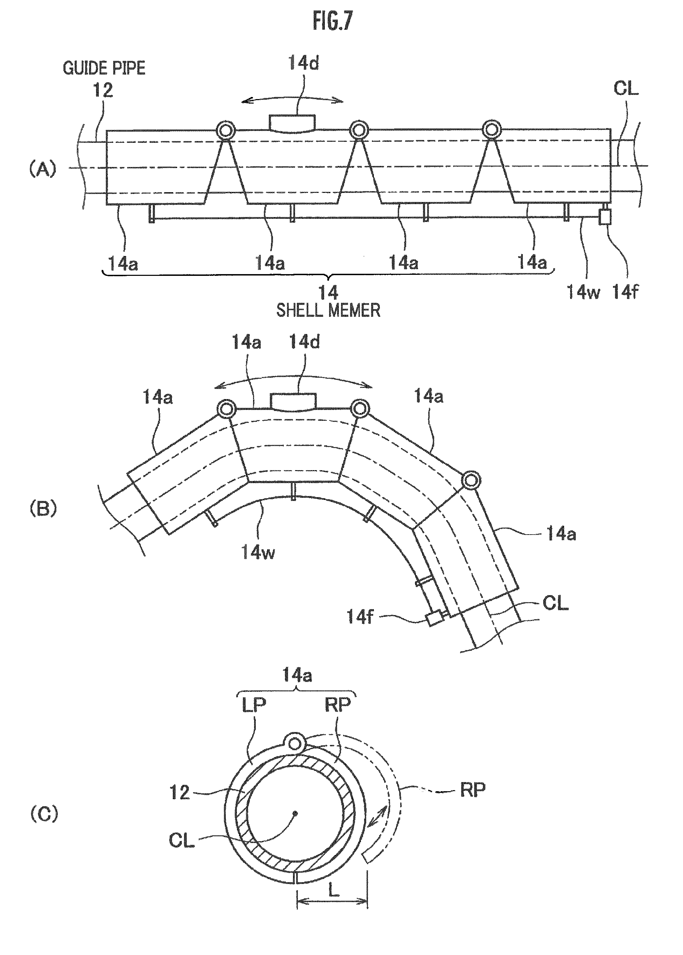

However, in the case where the guide pipe 12 having flexibility is used, if a shell member 14 as is shown in Fig. 7 is used as the supporting member 13, it is not necessary to remove and insert the shaft S of the endoscope each time the posture of the patient is changed, so that it becomes possible to promptly perform the examination and the treatment using the endoscope.

The shell member 14 will be explained hereinbelow.
As is shown in Fig. 7, the shell member 14 is a tubular member equipped with a through-hole penetrating between both ends thereof (in Fig. 7(A), between the right and left ends). The shell member 14 is formed by coupling a plurality of tubular members 14a, which is a member equipped with a through-hole penetrating in an axial direction (in Fig. 7(A), in the horizontal direction).
In Fig. 7, a reference 14d is a publicly known member for mounting the shell member 14 to the holding section 15 shown in Fig. 1 and Fig. 2.

As is shown in Fig. 7, a plurality of the tubular members 14a are connected so that the adjacent tubular members 14a are mutually flexible. Specifically, a plurality of the tubular members 14a are connected so that central axes of all tubular members 14a are positioned within an identical plane, and, so that adjacent tubular members 14a are mutually flexible in the state where the central axes of all tubular members 14a are positioned within the identical plane.
Further, a plurality of the tubular members 14a are connected so that, in the state where the shell member 14 is straight (the state shown in Fig. 7(A)), central axes of the through-holes of all the tubular members 14a are aligned on a same axis (on CL in Fig. 7(A), hereinafter referred to at a central axis CL of the shell member 14).

A method for connecting a plurality of the tubular members 14a is not particularly limited, as long as it is capable of mutually bending the adjacent tubular members 14a while maintaining the state mentioned above (the state where the central axes of all the tubular members 14a are positioned within the identical plane).
For example, as is shown in Fig. 7(A), adjacent tubular members 14a are connected at an axis orthogonal to the axial direction (a connecting axis), and connected so that all the connecting axes are mutually parallel and is positioned at a same side with respect to the central axis CL of the shell member 14. In this case, it becomes possible to mutually bend the adjacent tubular members 14a, in the state where the central axes of all the tubular members 14a are positioned within the identical plane.

Further, the shell member 14 is equipped with a posture holding means which holds the posture, that is, the bentness of the shell member 14. Any method may be used as the posture holding means, as long as it is capable of holding the posture of the shell member 14 from changing, even when a force seeking to straighten the shell member 14 is applied from the shaft S of the endoscope.

For example, as is shown in Fig. 7(A), in the case where the adjacent tubular members 14a are connected by the connecting axis orthogonal to the axis direction, then a wire 14w, and a hoisting device 14f which is capable of winding up and feeding of the wire 14w and is capable of holding the feed amount of the wire 14, such as a winch or a reel, may be adopted as the posture holding means.
In the case where the wire 14w and the hoisting device 14f are used, as is shown in Fig. 7(A), the hoisting device 14f is mounted to the tubular member 14a positioned at one end of the shell member 14, and a leading end of the wire 14w is fixed via a bracket and the like to the tubular member 14a positioned at the other end of the shell member 14. Also, the wire 14w is arranged so as to be positioned within the identical plane as the central axis CL of the shell member 14. Thereafter, the shell member 14 is set to a predetermined posture (refer to Fig. 7(B)), the hoisting device 14f winds up the wire 14w until the wire 14w is tensioned at this state, and maintains the state where the wire 14w is tensioned. By doing so, a tensile force of the wire 14w becomes a resistance force against the force seeking to straighten the shell member 14 from the shaft S of the endoscope, so that the shell member 14 may be maintained to the predetermined posture.

In the case where the shell member 14 mentioned above is used, by adjusting the bentness of the shell member 14 and fixing the bentness by the posture holding means, the through-hole of the shell member 14 may be made to a through-hole having a predetermined bentness.
Moreover, by deforming the shell member 14, it becomes possible to bend the shaft S of the endoscope along with the guide pipe 12 having flexibility. That is, by mounting the shell member 14, even when the posture of the patient is changed while the shaft S is inserted to the patient, it becomes possible to bend the shaft S in line with the change of the posture. As such, it is not necessary to insert the shaft S of the endoscope each time the posture of the patient is changed, it becomes possible to promptly perform the examination and treatment by the endoscope.

Further, the one having the structure as is shown in Fig. 7 is used as the posture holding means, when the wire 14w is wound up by the hoisting device 14f, the tensile force of the wire 14w may be used as a force for bending the shaft S of the endoscope, and it becomes possible to facilitate the operation of bending the shaft S of the endoscope.

Also, the tubular member 14a may be formed from a pair of wall members LP, RP having tubular inner surface, and the pair of wall members LP, RP may be capable of approaching to and departing from one another. In this case, the shell member 14 may be attached to the guide pipe 12 afterwards. This is preferable in that, in the state where there is a necessity to change the posture of the patient after the examination or the treatment is started, and the posture of the patient is changed while inserting the endoscope to the patient, it becomes possible to bend the endoscope in line with the change in the posture.

For example, in the case where the tubular member 14a is formed from the pair of wall members LP, RP formed in an arc-like cross section, one end edge in the wall member LP and RP are connected by an axis (a connecting axis) parallel to the central axis of the tubular member 14a. In this case, the other end edge of the wall member LP and RP are capable of swinging taking the connecting axis as a supporting point, so that when the other end edges are moved apart from each other, a gap is formed between the other end edges. If the length L of the gap is wider than the diameter of the guide pipe 12, it becomes possible to insert the guide pipe 12 to the through-hole of the tubular member 14a through this gap. That is, by moving apart the other end edges of the wall members LP, RP in all of the tubular members 14a, it becomes possible to attach the shell member 14 to the guide pipe 12 even when the endoscope is inserted into the patient.

It is preferable that the pair of wall members LP, RP are equipped with a mechanism for fixing the other end edges when the two are approached together. However, even the same is not equipped with a fixing mechanism, it is possible to maintain the state where the two are approached together, by a tape or a rope or the like.
Further, the pair of wall members LP, RP may be connected to each other as is explained above, but may be a structure in which the pair of wall members LP, RP becomes completely separated.

Moreover, in the above-mentioned example, explanation is given on the case where the shell member 14 is attached to the guide pipe 12. However, it goes without saying that, in the case where the guide pipe 12 is not used, the shell member 14 may be attached directly to the shaft S of the endoscope.

### (Fixing on face)

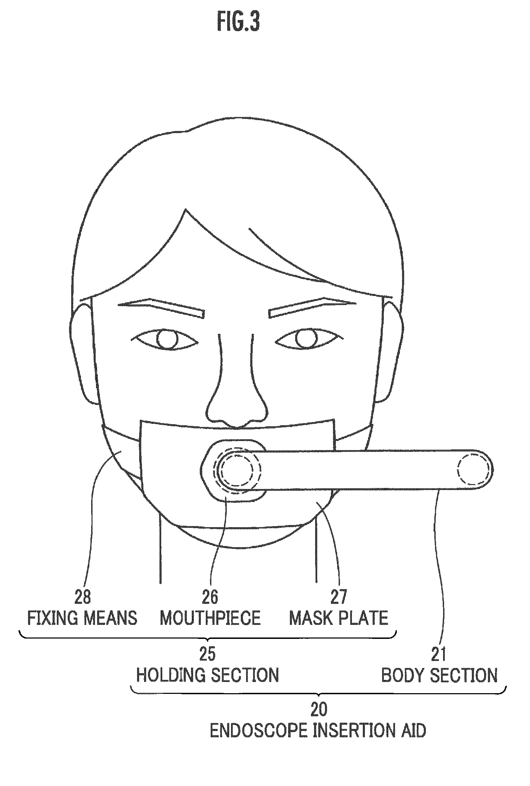

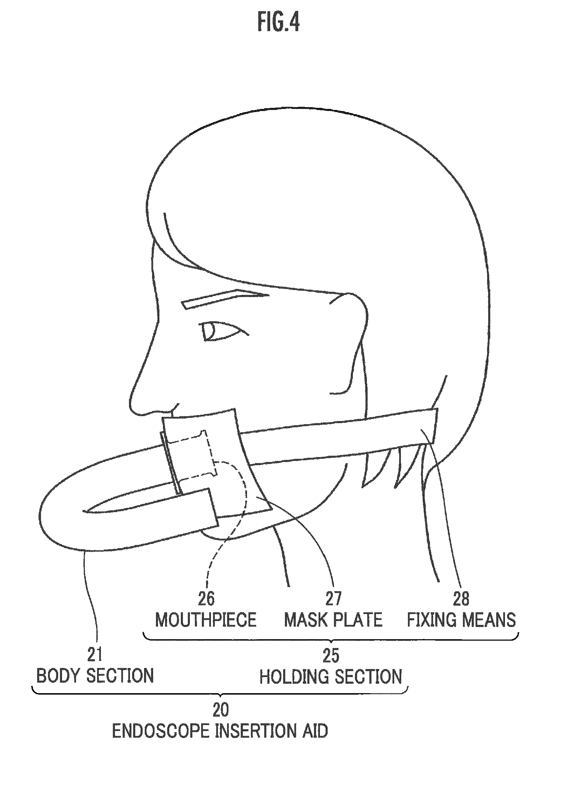

Further, in the above-mentioned example, explanation is given on the case where the holding section 15 has a structure of being fixed onto the bed B or the like. However, as is shown in Fig. 3 and Fig. 4, a body section 21 may be fixed to a face of a human via a holding section 25.
Hereinafter, explanation is given on an endoscope insertion aid 20 in which the body section 21 is fixed to the face of the human.

As is shown in Fig. 3 and Fig. 4, reference 21 denotes a body section which is a tubular member, and which is bent into a U-shape. As the body section 21, the one having similar structure and function as the body section 11 in the above-mentioned embodiments may be adopted.

As is shown in Fig. 3 and Fig. 4, reference 26 denotes a mouthpiece. The mouthpiece 26 is connected with one end of the body section 21. Also, between the mouthpiece 26 and the other end of the body section 21, a through-hole communicating the two is formed. That is, the bent through-hole penetrating the two is formed.
Therefore, when the shaft S of the endoscope is inserted from an opening on the other end of the body section 21, the shaft S may be introduced into the body of the patient while being guided by the through-hole.

Further, as is shown in Fig. 3 and Fig. 4, the mouthpiece 26 is fixed to a mask plate 27. The mask plate 27 is integrally formed from the same material as the mouthpiece 26. That is, the mask plate 27 is connected so that it is incapable of moving relatively to the mouthpiece 26.
Further, the mask plate 27 is a concaved surface concave to the side of the mouthpiece 26. The concaved surface is formed in a shape, in the state where the patient holds the mouthpiece 26 in his/her mouth, so that the concaved surface closely contacts the face of the patient.

As is shown in Fig. 3 and Fig. 4, the mask plate 27 is provided with a fixing means 28 for fixing the mask plate 27 to the head of the patient. The fixing means 28 is a publicly known fixing means such as a rubber band or a belt, which is capable of preventing the mask plate 27 from detaching even when the patient is not firmly holding the mouthpiece 26 in his/her mouth.

As is explained above, the endoscope insertion aid 20 may fix the mouthpiece 26 to the face of the patient with the mask plate 27 of a high intensity. Therefore, even when the shaft S of the endoscope is inserted into the through-hole of the body section 21, the force applied from the shaft S to the body section 21 may be supported by a jawbone and the like.
Therefore, even when the force is applied from the shaft S to the body section 21 during the operation of inserting the endoscope or the examination and treatment using the endoscope, it becomes possible to hold the body section 21 in a stable state.
Moreover, the force applied to the body section 21 is supported by large bones having high intensity such as the jawbone via the mask plate 27, the force applied to the teeth contacting the mouthpiece 26 and the like may be decreased.

In the above-mentioned example, explanation is given on the case where the mouthpiece 26 and the mask plate 27 are integrally formed, however, the two only need to be connected so that they cannot relatively move, and it is not necessary to form the two integrally. In the case where the two are not formed integrally, it becomes possible to form the mask plate 27 from material having the same or surpassing intensity as the material of the mouthpiece 26. By doing so, it becomes possible to improve the function of holding the mouthpiece 26 by the mask plate 27.

Also, one end of the body section 21 may be fixed to the mouthpiece 26, but one end of the body section 21 may be provided removably to the mouthpiece 26. In this case, it becomes possible to attach only the holding section 25 to the patient in the normal state, and use the body section 21 only when necessary. In this case, when the posture of the patient is changed, the examination and the treatment after posture change may be performed by removing and mounting the body section 21, the posture changing operation is made easy, and the examination and treatment may be started promptly after the posture change.

### (Embodiment without the holding section 15)

In the above-mentioned embodiment, explanation is given on the case where the endoscope insertion aid 10 supports the force applied to the body section 11 by the holding section 15. However, it is not necessary to provide the holding section 15.
For example, in the case where the force applied from the shaft S of the endoscope to the body section 11 may be supported only by a person holding (grasping) the body section 11, it becomes possible to insert the shaft S of the endoscope into the body of the patient, using the endoscope insertion aid 10 with only the body section 11 and without the holding section 15, to the patient in the right lateral recumbent position.
In this case, the time necessary until inserting the shaft S of the endoscope into the patient may be shortened to the extent that the holding section 15 is not fixed to the bed B and the like, and the posture of the patient is not limited by the position which the holding section 15 is fixed. Further, the endoscope insertion aid 10 may be used even in places where a target to which the holding section 15 is fixed does not exist, the place where the patient is laid (the place where the examination or the operation is performed) and the like is not limited.
In the case where the force applied from the shaft S of the endoscope to the body section 11 may be supported by the force of a human, it goes without saying that the holding section 15 of the endoscope insertion aid 10 may be held by a human, and the shaft S of the endoscope may be inserted into the body of the patient, to the patient in the right lateral recumbent position.

### Industrial Applicability

The endoscope insertion aid of the present invention is suitable for performing the examination and treatment, in the examination and treatment with the endoscope at a posture other than the left lateral recumbent position.

### Explanation of references

- 10:: Endoscope insertion aid
- 11:: Body section
- 11h:: Through-hole
- 12:: Guide pipe
- 13:: Supporting member
- 13a:: Supporting curved surface
- 14:: Shell member
- 14a:: Tubular member
- 15:: Holding section
- 20:: Endoscope insertion aid
- 21:: Body section
- 22:: Guide pipe
- 23:: Supporting member
- 25:: Holding section
- 26:: Mask plate
- 27:: Mask plate
- 28:: Fixing means
- S:: Shaft
- M:: Mouthpiece
- LP:: Wall member
- RP:: Wall member

## Claims

1. An endoscope insertion aid which supports an endoscope inserted to a mouthpiece held in a mouth of a patient, at outside a mouth cavity,
**characterized in that** the endoscope insertion aid is equipped with a body section equipped with a through-hole having an inner diameter capable of being inserted with the endoscope, and
the body section is capable of holding the endoscope in a bent state, in a state where the endoscope is inserted into the through-hole of the body section and thereafter inserted to the mouthpiece.

2. The endoscope insertion aid according to Claim 1,
**characterized in that** the body section is equipped with a holding section which fixes the body section so as not to move.

3. The endoscope insertion aid according to Claim 2,
**characterized in that** the through-hole formed on the body section is bent, and
the holding section is for fixing the body section so as not to move by a force applied from the endoscope when the endoscope is inserted into the through-hole of the body section.

4. The endoscope insertion aid according to any one of Claims 1, 2, or 3,
**characterized in that** the body section is equipped with a guide pipe which is a tubular member and which is bent between one end and the other end thereof.

5. The endoscope insertion aid according to Claims 3 or 4,
**characterized in that** the body section is equipped with the guide pipe, and a supporting member which supports a force applied from the endoscope to the guide pipe when the endoscope is inserted into the guide pipe, and
the supporting member is equipped with a concaved supporting curved surface which supports the force applied from the endoscope to the guide pipe.

6. The endoscope insertion aid according to any one of Claims 3, 4, or 5,
**characterized in that** the guide pipe is a tubular member formed in a U-shape.

7. The endoscope insertion aid according to Claim 1 or 2,
**characterized in that** the body section is equipped with a shell member formed with the through-hole and which is capable of deforming so that a central axis of the through-hole becomes a bent state between one end and the other end of the through-hole, and a posture holding means for holding the posture of the shell member.

8. The endoscope insertion aid according to Claim 7,
**characterized in that** the shell member is formed from a plurality of tubular members, and
a plurality of the tubular members are connected so that central axes of all tubular members are positioned within an identical plane, and, adjacent tubular members are mutually flexible in the state where the central axes of all tubular members are positioned within the identical plane.

9. The endoscope insertion aid according to Claim 7 or 8,
**characterized in that** the tubular member is equipped with a pair of wall members having a tubular inner surface, and
a pair of the wall members are configured so as to be capable of approaching and departing from one another, and so that the through-hole is formed between the two in the state where the two are approaching one another, and the endoscope is capable of being arranged between the two when the two are departing from one another.

10. The endoscope insertion aid according to any one of Claims 2 through 9,
**characterized in that** the body section is a tubular member,
the holding section is equipped with a mouthpiece to which one end of the body section is mounted,
a mask plate which is fixed with the mouthpiece, and which is mounted so as to cover a face of a patient, and
a fixing means for fixing the mask plate to a head of the patient, and the mask plate is formed from a material having same or surpassing intensity as the material of the mouthpiece.

11. The endoscope insertion aid according to Claim 10,
**characterized in that** one end of the body section is removable and attachable with respect to the mouthpiece of the holding section.
